(19)

**Europäisches Patentamt**
**European Patent Office**
**Office européen des brevets**

(11) **EP 4 223 282 A1**

(12) **EUROPEAN PATENT APPLICATION**

(43) Date of publication:
**09.08.2023 Bulletin 2023/32**

(21) Application number: **22382101.8**

(22) Date of filing: **08.02.2022**

(51) International Patent Classification (IPC):
**A61K 9/00** (2006.01)    **A61K 9/16** (2006.01)
**A61K 31/216** (2006.01)

(52) Cooperative Patent Classification (CPC):
**A61K 31/216; A61K 9/0019; A61K 9/1647**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
• **Servizo Galego de Saude**
  **15703 Santiago de Compostela (ES)**
• **Fundación Profesor Novoa Santos**
  **15006 A Coruña (ES)**
• **FUNDACION INSTITUTO DE INVESTIGACION SANITARIA DE**
  **SANTIAGO DE COMPOSTELA**
  **La Coruna (ES)**
• **Universidade de Santiago de Compostela**
  **15782 Santiago de Compostela (ES)**

(72) Inventors:
• **CARAMÉS PÉREZ, Beatriz**
  **15006 A Coruña (ES)**
• **BLANCO GARCÍA, Francisco Javier**
  **15006 A Coruña (ES)**
• **DOMÍNGUEZ MEDINA, Eduardo**
  **15782 Santiago de Compostela (ES)**
• **DÍAZ RODRÍGUEZ, Patricia**
  **15782 Santiago de Compostela (ES)**
• **NOGUEIRA RECALDE, Uxía**
  **15006 A Coruña (ES)**

(74) Representative: **Hoffmann Eitle**
  **Hoffmann Eitle S.L.U.**
  **Paseo de la Castellana 140, 3a planta**
  **Edificio LIMA**
  **28046 Madrid (ES)**

(54) **MICROSPHERES FOR EXTENDED RELEASE OF FENOFIBRATE**

(57)    The invention relates to biodegradable microspheres for extended release of fenofibrate, injectable formulations comprising said microspheres and their use for the treatment of joint related disorders, such as osteoarthritis. The invention furthermore relates to a method of preparing said microspheres.

EP 4 223 282 A1

## Description

### Technical field

The invention relates to biodegradable microspheres for extended release of fenofibrate, injectable formulations comprising said microspheres and their use for the treatment of joint related disorders, such as osteoarthritis. The invention furthermore relates to a method of preparing said microspheres.

### Background of the invention

Osteoarthritis is the most common form of arthritis. It is characterised by joint damage and joint failure, as the process of the disease causes damage to cartilage and the growth of new bone in affected joints, causing stiffness and pain. Osteoarthritis of the large joints, such as knee and hip reduce people's mobility and can make it difficult or impossible to climb stairs or walk. Osteoarthritis in small joints such as the hands and fingers make ordinary tasks difficult and painful. Ageing is a major risk factor in patients with cartilage degeneration and Osteoarthritis.

Fenofibrate is in clinical use for the treatment of lipid metabolism dysfunction and well known as a treatment option against high cholesterol levels. In recent studies fenofibrate was selected as a suitable candidate for a potential treatment against cartilage ageing and osteoarthritis (Nogueira-Recalde et al. 2019). In this study it was shown that osteoarthritis patients treated with fenofibrate have improved physical functions and therefore showed better mobility. This study provided novel evidence that activating peroxisome proliferator-activated receptor alpha (PPARα) by fenofibrate, a PPARa agonist, prevents cartilage degradation by modulating key mechanisms such as senescence and autophagy in chondrocytes and cartilage. The data established preclinical evidence that could lead to the development of novel disease-modifying therapies targeting lipid metabolism to prevent and treat osteoarthritis

However, the joint poses major challenges for pharmaceutical development. The synovium represents a barrier for penetration of drugs from plasma to the joint space, and the articular cartilage has no blood vessels and thus has limited access to systemically administered drugs. On the other hand, Fenofibrate is highly hydrophobic and insoluble in water and classified as a Biopharmaceutics Classification System class II compound because of its low solubility and limited gastrointestinal absorption rate. These characteristics represent a challenge for fenofibrate administration and entails a low synovial bioavailability in the joint. There is therefore a need for new formulations that are suitable for intraarticular injection that can overcome the problem of low bioavailability of the drug in the target tissue as well as the side effects caused by systemic administration.

Biodegradable microspheres made of polylactic co-glycolic acid copolymer (PLGA) are known in the field of drug delivery. PLGA is made of polylactic acid (PLA) and polyglycolic acid (PGA) and is an FDA-approved biodegradable polymer. It has been extensively investigated in many medical and pharmaceutical fields due to its good biodegradability and biocompatibility. PLGA-containing microspheres have shown sustained release characteristics due to degradation and diffusion mechanisms. The drug release profile of a PLGA microsphere preparation is dependent on certain factors, such as the specific properties of the drug, the ratio of PLA to PGA, the molecular weight/inherent viscosity of the polymer, the loading ratio of drug to the polymer and the size of the microspheres.

In 2017 Zilretta®, a PLGA-triamcinolone-acetonide microsphere formulation for knee injection, was approved by the FDA. Zilretta® exhibits pain reduction in knee osteoarthritis patients for up to 12 weeks.

Patients suffering from joint disorders and specifically osteoarthritis are in need of constant treatment to ease their discomfort. Daily intake of a drug is tedious and good patient compliance is necessary for positive treatment results. Furthermore, systemic delivery of a drug can lead to unwanted side effects. So far, no reliable long-term treatment options with fenofibrate are available in the market that could overcome these disadvantages. There is thus an unmet need for a superior way to treat joint disorders, such as osteoarthritis, using fenofibrate, while minimizing the drug's side effects observed with systemic delivery.

### Summary of the Invention

The present invention therefore relates to a biodegradable microsphere, wherein the microsphere

(i) comprises a polylactic-co-glycolic acid copolymer (PLGA) matrix, wherein said matrix comprises at least two PLGAs with different molecular weight (Mw);
(ii) has a diameter of from about 25 μm to about 110 μm;
(iii) comprises fenofibrate; and
(iv) when present in the target tissue, releases fenofibrate for at least one month.

In one embodiment the at least two PLGAs each have a viscosity from about 0.16 dl/g to about 1.70 dl/g, preferably wherein one of the said at least two PLGAs has a viscosity from about 0.50 dl/g to about 0.70 dl/g and the second one of the at least two PLGAs has a viscosity from about 0.16 dl/g to about 0.24 dl/g.

In a further embodiment the at least two PLGAs have a Mw between about 6 kDa and about 90 kDa, preferably wherein one of the said at least two PLGAs has a Mw of between about 6 kDa and about 18 kDa and the second one of the at least two PLGAs has a Mw of between about 50 kDa and about 90 kDa.

In one embodiment one of the said at least two PLGAs has an average lactic acid to glycolic acid molar ratio from 100:0 to 50:50, preferably wherein one of the at least two PLGAs has an average lactic acid to glycolic acid molar ratio of 75:25 (PLGA 75:25) and the second one of the at least two PLGAs has an average lactic acid to glycolic acid molar ratio of 50:50 (PLGA 50:50).

In a further embodiment the ratio between PLGA 50:50 and PLGA 75:25 is selected from about 90:10, about 80:20 or about 75:25, preferably from about 90:10.

In a further embodiment the diameter of the microsphere is between about 25 $\mu$m to about 110, about 30 $\mu$m to about 100 $\mu$m, about 40 $\mu$m to about 90 $\mu$m, about 45 $\mu$m to about 80 $\mu$m, about 50 $\mu$m to about 70 $\mu$m, most preferred about 55 $\mu$m to about 65 $\mu$m.

In one preferred embodiment the microsphere comprises from about 0.5 $\mu$g to about 20 $\mu$g, preferably from about 0.8 $\mu$g to about 15 $\mu$g, most preferred from about 1 $\mu$g to about 10 $\mu$g of fenofibrate per milligram of microsphere.

In a preferred embodiment the microsphere

(i) comprises a polylactic-co-glycolic acid copolymer (PLGA) matrix, wherein said matrix comprises PLGA 75:25 with a viscosity of about 0.60 dl/g and/or a Mw of about 68 kDa and PLGA 50:50 with a viscosity of about 0.21 dl/g and/or a Mw of about 13.5 kDa

(ii) has a diameter of about 60 $\mu$m;

(iii) comprises 1 $\mu$g to 10 $\mu$g fenofibrate per milligram of microsphere, preferably 10 $\mu$g fenofibrate per milligram of microsphere; and

(iv) when present in the target tissue, releases fenofibrate for at least two months, more preferably for at least three months.

In a further aspect the present invention relates to a plurality of biodegradable microspheres as defined in any one of the preceding claims, wherein the microspheres have a

(i) d90 particle size value between about 50 $\mu$m and about 100 $\mu$m; and/or

(ii) d10 particle size values between about 16 $\mu$m and about 20 $\mu$m; and/or

(iii) d50 particle size values between about 30 $\mu$m and about 60 $\mu$m.

In a preferred embodiment of the plurality of biodegradable microspheres the microspheres

(i) have a d90 particle size value from about 50 $\mu$m to about 100 $\mu$m;

(ii) comprise a polylactic-co-glycolic acid copolymer (PLGA) matrix, wherein said matrix comprises at least two PLGAs with different molecular weight (Mw);

(iii) carry a therapeutically effective amount of fenofibrate; and

(iv) when present in the target tissue, release fenofibrate for at least one month.

In a further preferred embodiment the at least two PLGAs each have a viscosity from about 0.16 dl/g to about 1.70 dl/g, preferably wherein one of the said at least two PLGAs has a viscosity from about 0.50 dl/g to about 0.70 dl/g and the second one of the at least two PLGAs has a viscosity from about 0.16 dl/g to about 0.24 dl/g.

In yet another embodiment of the plurality of biodegradable microspheres the at least two PLGAs have a Mw between about 6 kDa and about 90 kDa, preferably wherein one of the said at least two PLGAs has a Mw of between about 6 kDa and about 18 kDa and the second one of the at least two PLGAs has a Mw of between about 50 kDa and about 90 kDa.

In a further embodiment of the plurality of biodegradable microspheres one of the said at least two PLGAs has an average lactic acid to glycolic acid molar ratio from 100:0 to 50:50, preferably wherein one of the at least two PLGAs has an average lactic acid to glycolic acid molar ratio of 75:25 (PLGA 75:25) and the second one of the at least two PLGAs has an average lactic acid to glycolic acid molar ratio of 50:50 (PLGA 50:50).

In a preferred embodiment the ratio between PLGA 50:50 and PLGA 75:25 is selected from about 90:10, about 80:20 or about 75:25, preferably from about 90:10.

In one embodiment the diameter of the microsphere is between about 25 $\mu$m to about 110 $\mu$m, about 30 $\mu$m to about 100 $\mu$m, about 40 $\mu$m to about 90 $\mu$m, about 45 $\mu$m to about 80 $\mu$m, about 50 $\mu$m to about 70 $\mu$m, most preferred about 55 $\mu$m to about 65 $\mu$m.

In one embodiment the microspheres comprise from about 0.5 μg to about 20 μg, preferably from about 0.8 μg to about 15 μg, most preferred from about 1 μg to about 10 μg of fenofibrate per milligram of microsphere.

The present invention furthermore relates to an injectable formulation comprising a pharmaceutically acceptable carrier and the microspheres or a plurality of biodegradable microspheres as described herein.

In a preferred embodiment of said formulation the microspheres, when present in the target tissue, release fenofibrate for at least one month, preferably for at least two months, more preferably for at least three months.

The present invention furthermore relates to the biodegradable microsphere, the plurality of biodegradable microspheres, or the injectable formulation as described herein above for use in the treatment or prevention of a joint-related disorder, preferably arthritis, more preferably osteoarthritis.

In one embodiment the biodegradable microsphere, the plurality of biodegradable microspheres, or the injectable formulation as described herein is administered intra-articularly, preferably wherein said microspheres, plurality of microspheres, or injectable formulation is administered as a single dose.

The present invention furthermore relates to a kit comprising, in separate compartments,

(a) a diluent, and
(b) biodegradable microspheres, or a plurality of biodegradable microspheres according to any one of the preceding claims.

In a preferred embodiment the said biodegradable microspheres, or plurality of biodegradable microspheres is present in powder form.

The present invention furthermore relates to a method for preparing the microspheres as described herein, wherein the method comprises single emulsion-solvent evaporation. In a preferred embodiment of said method the microspheres are prepared with polymeric concentrations from about 10 - 30 % (w/w), preferably 15 - 25% (w/w), more preferably 15-20 % (w/w) of PLGA matrix.

In one embodiment of said method at least 90% of the fenofibrate are encapsulated into the microspheres.

The present invention furthermore relates to a method for treating a joint-related disorder in a subject comprising introducing the biodegradable microspheres, the plurality of biodegradable microspheres, or the injectable formulation as described herein into target tissue in or around one or more of the subject's joints, preferably the hips, shoulders and/or knees.

In one embodiment the subject is a human or an animal, such as a cat, a dog, or a horse.

In a further embodiment the joint-related disorder is arthritis, preferably osteoarthritis.

In one embodiment said method comprises intra-articularly injecting the biodegradable, the plurality of biodegradable microspheres, or the injectable formulation into one or both of the subject's knees, shoulders, hips, neck, lower back, small joints of the hand or feet.

## BRIEF DESCRIPTION OF THE FIGURES

Figure 1: Fenofibrate loading on the different formulations obtained with a single PLGA (Example 1). The theoretical drug amount added was 1% of fenofibrate in the PLGA matrix (10μg fenofibrate/mg PLGA).

Figure 2: Fenofibrate release profile of different microsphere formulations obtained with a single PLGA as prepared in Example 1 over a total period of 90 days.

Figure 3: Particle size distribution of microspheres as prepared in Example 1.

Figure 4: Fenofibrate loading of formulations with specific MWs and optimized polymer ratios as prepared in Example 2. Encapsulation efficiency is achieved independently of the PLGA formulation selected. The theoretical drug amount added was 1% of fenofibrate in the PLGA matrix (10μg fenofibrate/mg PLGA).

Figure 5: Fenofibrate controlled release profile of different microsphere formulations prepared with PLGAs mixtures. PLGA (50:50) 13.5 kDa (0.21 dl/g) and PLGA (75:25) 68kDa (0.60 dl/g) (Example 2).

Figure 6: Particle size distribution of microspheres as prepared in Example 2.

Figure 7: Fenofibrate administered intra-articularly reduces joint damage in a preclinical model of Osteoarthritis in mice. Three-months-old C57Bl/6J mice were subjected to osteoarthritis surgery (MMTL+MCL) in the right knee.

The experiment included 24 mice (8 mice/each group). (A) Knee joints were analysed by staining with Safranin O. (B) Histological scores. Values are mean ± SEM. **$p<0.01$ versus PLGA 90:10 condition. Magnification: 10x

Figure 8: Fenofibrate administered intra-articularly reduces joint damage in a preclinical model of Osteoarthritis in mice. Three-months-old C57Bl/6J mice were subjected to osteoarthritis surgery (LMTL+LCL) in the right knee. The experiment included 24 mice (8 mice/each group). (A) Knee joints were analysed by staining with Safranin O. (B) Histological scores. Values are mean ± SEM. *$p<0.05$ versus PLGA 90:10 condition. Magnification: 10x

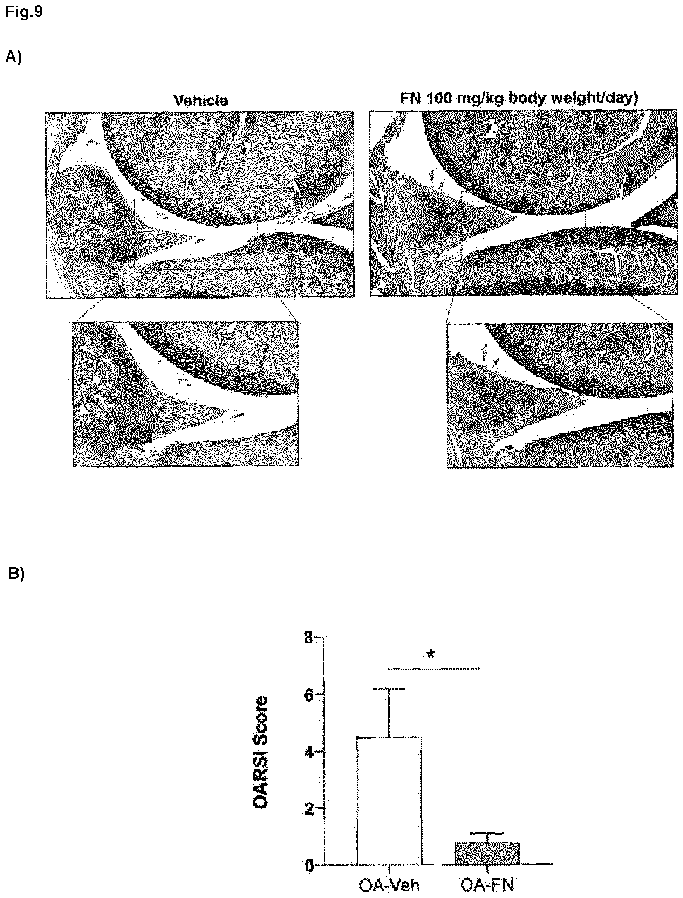

Figure 9: Oral administration of fenofibrate reduces joint damage in a preclinical model of Osteoarthritis in mice. Three-months-old C57Bl/6J mice were subjected to OA surgery in the right knee. The experiment included 16 mice (8 mice/each group). (A) Knee joints were analysed by staining with Safranin O. (B) Histological scores. Values are mean ± SEM. *$p<0.05$ versus Vehicle condition.

## Detailed description

The present invention may be understood more readily by reference to the following detailed description of the preferred embodiments of the invention, and to the examples included therein.

As described in more detail above there is an unmet need for an improved long-term option to treat joint disorders, such as osteoarthritis, using fenofibrate, while minimizing the drug's side effects observed with systemic delivery.

The inventors have therefore developed biodegradable microspheres comprising fenofibrate that overcome these disadvantages by providing an extended release profile in the target tissue without reducing the treatment effect compared to fenofibrate administered orally on a daily basis. The microspheres of present invention as well as their application are discussed in further detail here below.

## MICROSPHERES OF PRESENT INVENTION

Polylactic co-glycolic acid copolymer PLGA is an FDA-approved biodegradable copolymer made of polylactic acid (PLA) and polyglycolic acid (PGA). It has been extensively investigated in many medical and pharmaceutical fields due to its good biodegradability and biocompatibility. PLGA-containing microspheres can show sustained release characteristics due to degradation and diffusion mechanisms. However, the drug release profile of a PLGA microsphere preparation is highly dependent on certain factors, such as the specific properties of the drug itself, the ratio of PLA to PGA, the molecular weight/inherent viscosity of the polymer, the loading ratio of drug to the polymer, and the size of the microspheres. In general, low molecular weight and correspondingly low inherent viscosity PLGA is known to allow for more complete and faster release of pharmaceutical agents incorporated into microparticles than their higher molecular weight and higher inherent viscosity counterparts.

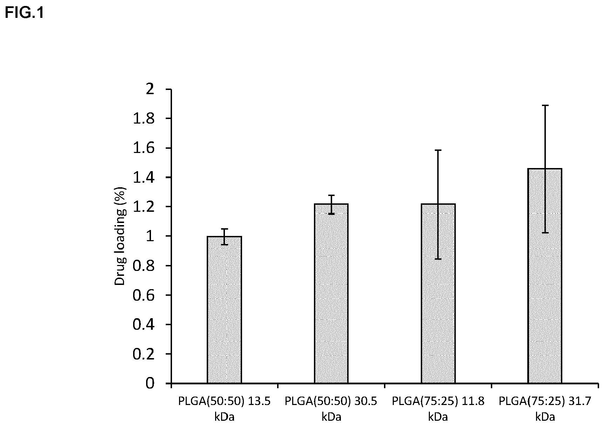

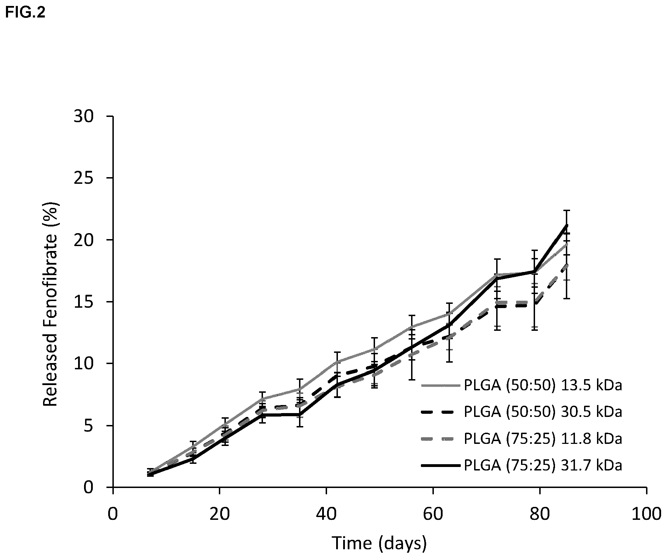

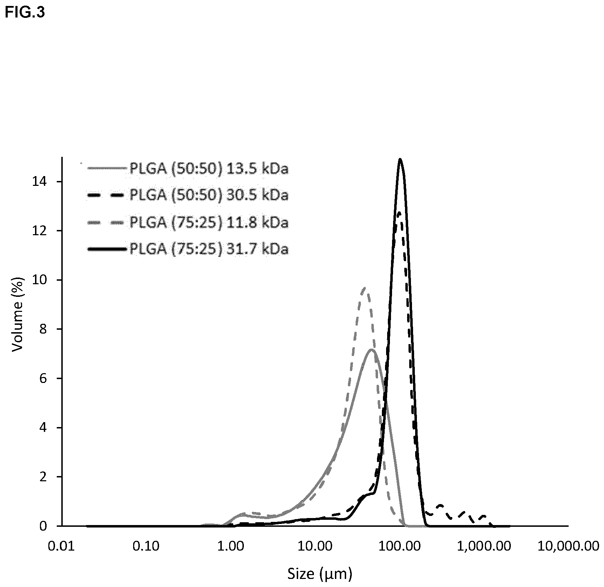

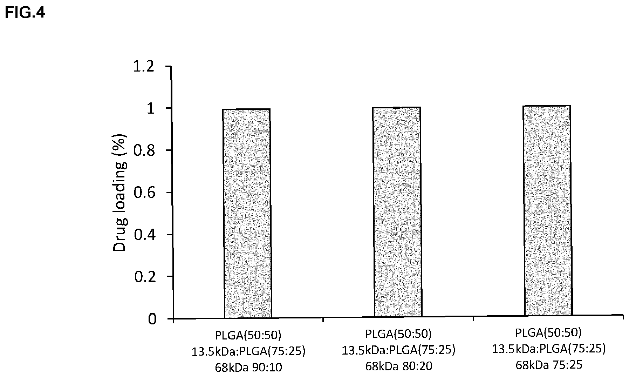

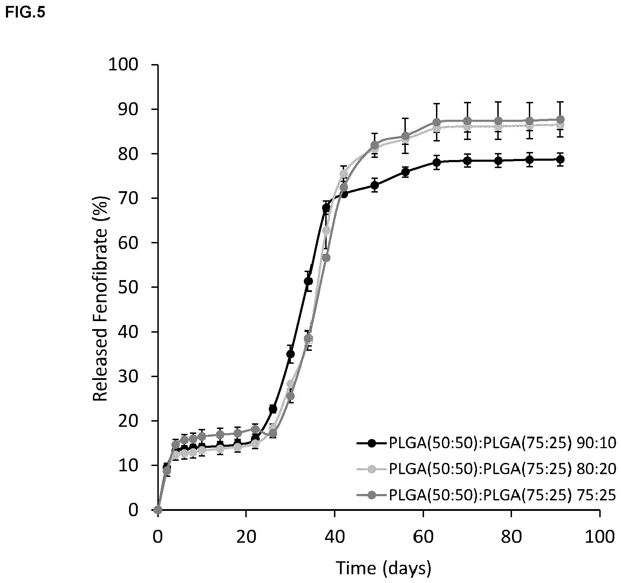

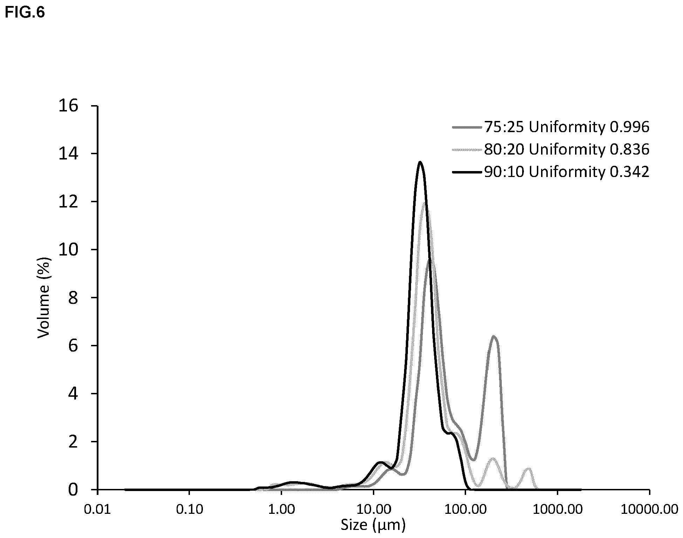

In order to find the ideal composition for the formation of microspheres that not only are able to encapsulate a substantial amount of fenofibrate, but also show the desired extended release profile over a certain time of the drug, the inventors have tested different compositions including single PLGAs and mixtures of variable PLGAs with variable L/G ratio and molecular weight. The data obtained when a single PLGA was used (Example 1) showed adequate encapsulation efficiency (Figure 1) and a controlled release profile reaching around 20% of the drug released after 3 months (Figure 2)The microspheres size and distribution was clearly dependent on the molecular weight of the polymer obtaining higher mean diameters for those microspheres prepared with PLGAs of medium molecular weight (Figure 3). The reduced particle size with decrease in polymer Mw is a result of reduced inherent polymer viscosity as the polymer concentration was constant in each formulation. Therefore, the microspheres with low Mw PLGA fulfilled the requirements for high encapsulation efficiency and morphology. However, it was found that their release rate was inefficient as the amount of released drug was not sufficient for providing the desired treatment efficacy. Therefore, to modulate the release rate while at the same time ensuring the morphological properties, a mixture of PLGAs of high and low molecular weight was used to prepare the microspheres (Example 2). These microspheres were characterized by a high encapsulation efficiency (Figure 4) and a controlled release profile exhibiting the typical triphasic release profile (Figure 5). The increase in burst release on the microspheres of blended PLGAs can be explained by the polymer miscibility resulting in microspheres with lower density and increased drug diffusion. These profiles provide a higher release rate with 80% of the drug released after three months allowing to reach the therapeutic concentration in the target tissue necessary to provide treatment efficacy. On the other hand, the microparticles mean size and distribution was clearly depended on the amount of PLGA of high Mw used (Figure 6). Microspheres with a very narrow size distribution are obtained when the low Mw (PLGA 50:50 13.5kDa):high MW (PLGA 75:25 68kDa) ratio used is of 90:10. Whilst the other two ratios tested also provided for a reasonably narrow size distribution, the preferred microspheres were the ones with the ratio of 90:10.

The present invention therefore relates to a biodegradable microsphere, wherein the microsphere

(i) comprises a polylactic-co-glycolic acid copolymer (PLGA) matrix, wherein said matrix comprises at least two PLGAs with different molecular weight (Mw);
(ii) has a diameter of from about 25 $\mu$m to about 110 $\mu$m;
(iii) comprises fenofibrate; and
(iv) when present in the target tissue, releases fenofibrate for at least one month.

In one embodiment the at least two PLGAs each have a viscosity from about 0.16 dl/g to about 1.70 dl/g. In a preferred embodiment one of the said at least two PLGAs has a viscosity from about 0.50 dl/g to about 0.70 dl/g, from about 0.52 dl/g to about 0.68 dl/g, from about 0.54 dl/g to about 0.66 dl/g, from about 0.56 dl/g to about 0.66 dl/g, from about 0.58 dl/g to about 0.64, from about 0.60 dl/g to about 0.62 dl/g, and the second one of the at least two PLGAs has a viscosity from about 0.16 dl/g to about 0.24 dl/g, from about 0.18 dl/g to about 0.22 dl/g, from about 0.20 dl/g to about 0.22 dl/g.

In a further embodiment the at least two PLGAs have a Mw between about 6 kDa and about 90 kDa, between about 10 kDa and 80 kDa, or between about 12 kDa and 70 kDa.

In a further preferred embodiment one of the said at least two PLGAs has a Mw of between about 6 kDa and about 18 kDa, between about 7 kDa and about 17 kDa, between about 8 kDa and about 16 kDa, between about 9 kDa and about 15 kDa, between about 10 kDa and about 14 kDa, and the second one of the at least two PLGAs has a Mw of between about 50 kDa and about 90 kDa, between about 55 kDa and about 80 kDa, between about 60 kDa and about 70 kDa.

In one embodiment the second one of the at least two PLGAs has a Mw of between about 190 to 240 kDa, preferably of about 215 kDa, or a Mw of between 76 kDa and 115 kDa, preferably 95.5 kDa.

In one embodiment one of the said at least two PLGAs have an average lactic acid to glycolic acid molar ratio of from about 100:0 to about 40:60, from about 90:10 to about 40:60, from about 80:20 to about 50:50, preferably wherein one of the at least two PLGAs has an average lactic acid to glycolic acid molar ratio of from about 75:25 (PLGA 75:25) and the second one of the at least two PLGAs has an average lactic acid to glycolic acid molar ratio of about 60:40 to about 40:60, preferably about 50:50 (PLGA 50:50).

To assess the preferred ratio in the microspheres to be used of PLGA 50:50 and PLGA 75:25, microspheres of three different ratios were prepared as described in Example 2. In a further embodiment the ratio between PLGA 50:50 and PLGA 75:25 is therefore selected from about 90:10, about 80:20 or 75:25, preferably from about 90:10, since this ratio shows adequate encapsulation efficiency and drug release profiles while ensuring the required microspheres size diameter and narrow size distribution (as shown in Fig.6).

In a further embodiment the diameter of the microsphere is between about 25 $\mu$m to about 110 $\mu$m, about 30 $\mu$m to about 100 $\mu$m, about 40 $\mu$m to about 90 $\mu$m, about 45 $\mu$m to about 80 $\mu$m, about 50 $\mu$m to about 70 $\mu$m, most preferred about 55 $\mu$m to about 65 $\mu$m. It is understood that these ranges refer to the mean diameter of all microparticles in a given population. The diameter of any given individual microparticle could be within a standard deviation above or below the mean diameter.

The drug loading capacity of the microspheres of present invention was assessed as described in Example 1. In one preferred embodiment the microsphere comprises from about 0.5 $\mu$g to about 15 $\mu$g, preferably from about 1 $\mu$g to about 10 $\mu$g of fenofibrate per milligram of microsphere.

In one embodiment, the fenofibrate contained in the microspheres is 0.1 - 1 % (w/w) of the microspheres, for example, between 0.2 - 0.9 % (w/w), between 0.3 - 0.8 % (w/w), between 0.4 - 0.7 % (w/w), between 0.4 - 0.6 % (w/w), of the microparticle.

In a preferred embodiment the microsphere

(i) comprises a polylactic-co-glycolic acid copolymer (PLGA) matrix, wherein said matrix comprises PLGA 75:25 with a viscosity of about 0.60 dl/g and/or a Mw of about 68 kDa and PLGA 50:50 with a viscosity of about 0.21 dl/g and/or a Mw of about 13.5 kDa
(ii) has a diameter of about 60 $\mu$m;
(iii) comprises 1 $\mu$g to 10 $\mu$g fenofibrate per milligram of microsphere, preferably 10 $\mu$g fenofibrate per milligram of microsphere; and
(iv) when present in the target tissue, releases fenofibrate for at least two months, more preferably for at least three months.

In a further aspect the present invention relates to a plurality of biodegradable microspheres as defined in any one of the preceding claims, wherein the microspheres have a

(i) d90 particle size value between about 50 $\mu$m and about 100 $\mu$m; and/or
(ii) d10 particle size values between about 16 $\mu$m and about 20 $\mu$m; and/or

(iii) d50 particle size values between about 30 μm and about 60 μm.

In a preferred embodiment of the plurality of biodegradable microspheres the microspheres

(i) have a d90 value from about 50 μm to about 100 μm;
(ii) comprise a polylactic-co-glycolic acid copolymer (PLGA) matrix, wherein said matrix comprises at least two PLGAs with different molecular weight (Mw);
(iii) carry a therapeutically effective amount of fenofibrate; and
(iv) when present in the target tissue, releases fenofibrate for at least one month.

As used herein, by "d90 particle size" is meant that the particle size distribution is such that 90% of the particles have a particle size diameter of less than the specified value.

As used herein, by "d50 particle size" is meant that the particle size distribution is such that 50% of the particles have a particle size diameter of less than the specified value.

As used herein, by "d10 particle size" is meant that the particle size distribution is such that 10% of the particles have a particle size diameter of less than the specified value.

The d10, d50 and d90 values may be determined by well-known methods of the prior art such as sieve analysis, laser diffraction methods, photoanalysis or optical counting methods. The particle size distribution was determined as described in Example 1 by light diffraction employing Mastersizer 2000. The particle size distribution of the microspheres comprising a PLGA 50:50 and PLGA 75:25 ratio from about 90:10, about 80:20 or about 75:25 was assessed as described in the example. As can be seen in Fig.6, whilst all three microparticles show a sufficiently sharp peak, the microspheres comprising 90:10 provide the most beneficial particle size distribution. As gathered from the uniformity value that range from 0 (where all the microspheres show exactly the same size) to 1 (where all the microspheres are different in size) the 90:10 formulation showed the most adequate distribution with a uniformity value of 0.342. Whilst the microspheres comprising the ratio of 80:20 and 75:25 were also suitable, the 90:10 formulation was considered the preferred one for the preparation of the fenofibrate microspheres.

d90 particle size ranging from about 50 μm to about 100 μm include values of about 45 μm, 50 μm, 55 μm, 60 μm, 70 μm, 75 μm, 80 μm, 85 μm, 90 μm, 95 μm, 100 μm and 105 μm.

d50 particle size values ranging from about 10 μm to about 30 μm include values of about 8 μm, 9 μm, 10μm, 11 μm, 12 μm, 13 μm, 14 μm, 15 μm, 16 μm, 18 μm, 19 μm, 20 μm, 21 μm, 22 μm, 23μm, 24 μm, 25 μm, 26 μm, 27 μm, 28 μm, 29 μm, 30 μm, 31 μm, 32 μm.

d10 particle size values between about 16 μm and about 20 μm include d10 particle size values of about 14 μm, 15 μm 16 μm, 17 μm, 18μm, 18 μm, 19μm, 20.0 μm, 21 μm, 22 μm.

In a further preferred embodiment, the at least two PLGAs each have a viscosity from about from about 0.16 dl/g to about 1.70 dl/g. In a preferred embodiment one of the said at least two PLGAs has a viscosity from about 0.50 dl/g to about 0.70 dl/g, from about 0.52 dl/g to about 0.68 dl/g, from about 0.54 dl/g to about 0.66 dl/g, from about 0.56 dl/g to about 0.66 dl/g, from about 0.58 dl/g to about 0.64, from about 0.60 dl/g to about 0.62 dl/g, and the second one of the at least two PLGAs has a viscosity from about 0.16 dl/g to about 0.24 dl/g, from about 0.18 dl/g to about 0.22 dl/g, from about 0.20 dl/g to about 0.22 dl/g.

In yet another embodiment of the plurality of biodegradable microspheres the at least two PLGAs have a Mw between about 6 kDa and about 90 kDa, between about 10 kDa and 80 kDa, or between about 12 kDa and 70 kDa.

In a further preferred embodiment one of the said at least two PLGAs has a Mw of between about 6 kDa and about 18 kDa, between about 7 kDa and about 17 kDa, between about 8 kDa and about 16 kDa, between about 9 kDa and about 15 kDa, between about 10 kDa and about 14 kDa, and the second one of the at least two PLGAs has a Mw of between about 50 kDa and about 90 kDa, between about 55 kDa and about 80 kDa, between about 60 kDa and about 70 kDa.

In a further embodiment one of the said at least two PLGAs has a Mw of between about 6 kDa and about 18 kDa, between about 7 kDa and about 17 kDa, between about 8 kDa and about 16 kDa, between about 9 kDa and about 15 kDa, between about 10 kDa and about 14 kDa, and the second one of the at least two PLGAs has a Mw of between about 25 kDa and about 40 kDa, between about 30 kDa and about 35 kDa.

In a further embodiment of the plurality of biodegradable microspheres one of the said at least two PLGAs has an average lactic acid to glycolic acid molar ratio of from about 100:0 to about 40:60, from about 90:10 to about 40:60, from about 80:20 to about 50:50, preferably wherein one of the at least two PLGAs has an average lactic acid to glycolic acid molar ratio of from 75:25 (PLGA 75:25) and the second one of the at least two PLGAs has an average lactic acid to glycolic acid molar ratio of 50:50 (PLGA 50:50).

In a preferred embodiment the ratio between PLGA 50:50 and PLGA 75:25 is selected from about 90:10, about 80:20 or 75:25, preferably from about 90:10.

In one embodiment the diameter of the microsphere is between about 25 μm to about 110 μm, 30 μm to about

100 μm, about 40 μm to about 90 μm, about 45 μm to about 80 μm, about 50 μm to about 70 μm, most preferred about 55 μm to about 65 μm.

In one embodiment the microspheres comprise from 0.5 μg to about 20 μg, preferably from about 0.5 μg to about 15 μg, p most preferred from about preferably from 1 μg to about 10 μg of fenofibrate per milligram of microsphere.

PHARMACEUTICAL COMPOSITIONS

The present in invention also relates to an injectable formulation comprising a pharmaceutically acceptable carrier and the microspheres or a plurality of biodegradable microspheres as described herein.

The injectable formulations of present invention and their efficacy of extended release of fenofibrate in microspheres were assessed as described in Example 2. Microspheres prepared with a single PLGA showed a sustained controlled and extended release of the drug for three months (see Fig.2). However, the percentage of drug release was relatively low for all the formulations with around 20% of the loaded drug released at this time point. Therefore, it was decided to combine the 50:50 PLGA of low molecular weight with a 75:25 PLGA of higher molecular weight to change the polymeric matrix structure in order to prepare microspheres that show a higher total release of the drug. As shown in Figure 5, the obtained microspheres led to a highest drug burst release followed by a drug diffusion-PLGA erosion-controlled drug release reaching around a 90% of the drug released after 3 months. This initial burst release is useful for reaching the required therapeutic concentration of the drug while the later controlled release profile allows to keep the drug concentration inside the therapeutic window.

In a preferred embodiment of said formulation the microspheres, when present in the target tissue show an extended release profile releasing fenofibrate for at least one month, preferably for at least two months, more preferably for at least three months.

In a further preferred embodiment, the total release of the drug from the microsphere is at least 60%, at least 70%, and preferably at least 80% after 50 days, preferably after 40 days. In one preferred embodiment the total release of the drug from the microsphere is at least 80%, at least 85 %, at least 90% after 60 days, preferably after 70 days, more preferred after 80 days and most preferred after 90 days.

USES AND TREATMENT

The present invention furthermore relates to the biodegradable microsphere, the plurality of biodegradable microspheres, or the injectable formulation as described herein above for use in the treatment or prevention of a joint-related disorder, preferably arthritis, more preferably osteoarthritis.

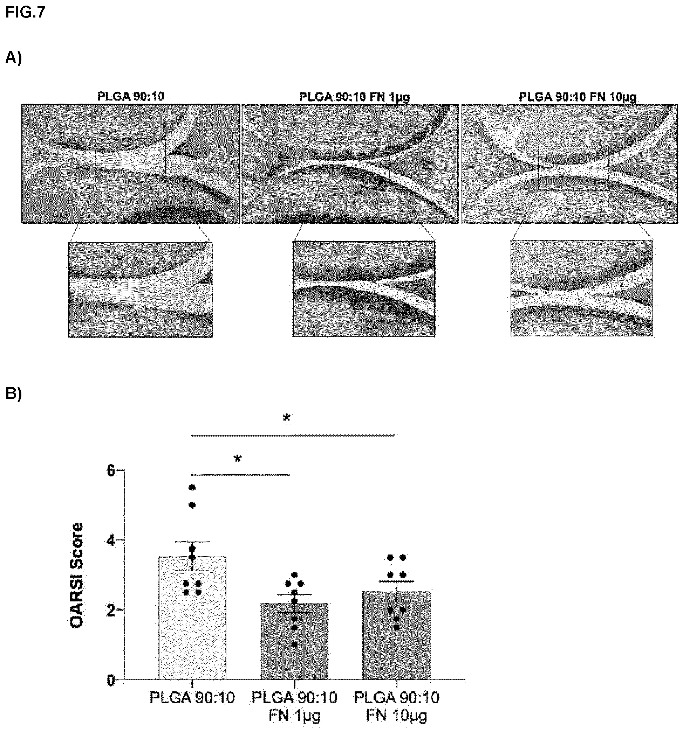

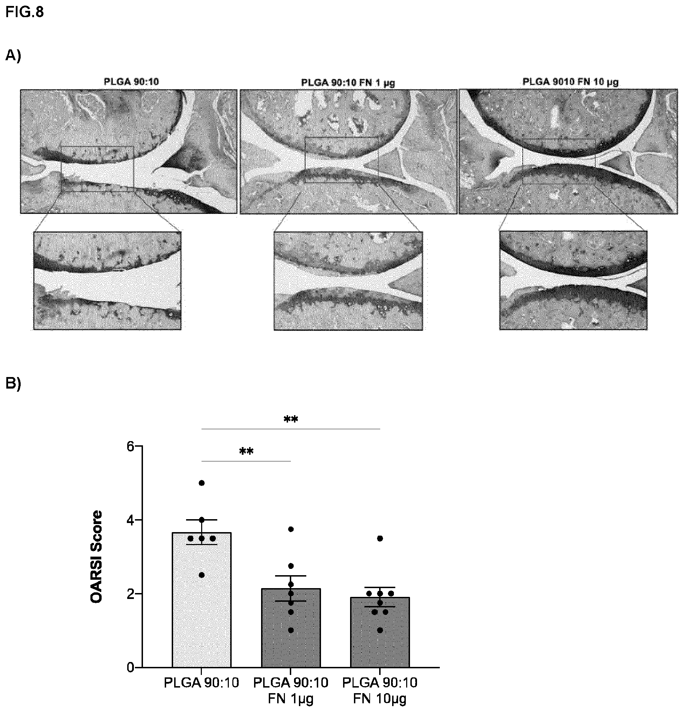

The injectable formulations of present invention and their efficacy of extended release of fenofibrate in microspheres by intra-articular injection were assessed in two independent preclinical models of surgically induced Osteoarthritis in mice as further detailed in Example 3 and Example 4. As can be seen in Fig.7 A), mouse knee joints in the vehicle group (PLGA 90:10) exhibited significant cartilage damage, including proteoglycan depletion, loss of surface lamina and cartilage fibrillations. However, PLGA 90:10 FN 1ug and PLGA 90:10 FN 10ug treatment significantly decreased the severity of these OA-like changes. Analysis of OA pathology by a semiquantitative scoring system indicated a significant decrease in the severity of the OA-like changes after extended release of fenofibrate treatment ($P < 0.01$) compared to vehicle group (Fig.7 B). As can be seen in Fig.8 A), mouse knee joints in the vehicle group (PLGA 90:10) exhibited significant joint damage. However, PLGA 90:10 FN 1ug and PLGA 90:10 FN 10ug treatment significantly decreased the severity of experimental OA. Analysis of OA pathology by a semiquantitative scoring system indicated a significant decrease in the OA severity after extended release of fenofibrate treatment ($P < 0.05$) compared to vehicle group (Fig.8 B).

As discussed above, patients suffering from joint disorders and specifically osteoarthritis are in need of constant treatment to ease their discomfort. One way of easing the pain for a longer period of time is the long-term daily intake of a drug, which can result tedious and requires good patient compliance for positive treatment results. Another disadvantage of administering a daily dose of a drug, which usually is done via an oral dosage from and thus systemically is the fact that systemic delivery of a drug can lead to unwanted side effects. The local administration at the joint of concern is therefore much preferred. In the case of fenofibrate, the drug can be administered via injection intra-articularly to avoid the systemic administration. However, in this case it is necessary that the injection can be given once, but provides a longer-term treatment, as it is not feasible for patients to get a daily injection into the joint. The inventors have therefore developed the herein described microspheres, as well as an injection formulation comprising said microspheres to provide the possibility of a one-time injection with a controlled and extended release of the active ingredient fenofibrate into the joint, thus overcoming the above disadvantages. Since to date no reliable long-term treatment options with fenofibrate are available in the market, the inventors herein provide a solution to this unmet need by providing a superior way to treat joint disorders, such as osteoarthritis, using fenofibrate, while minimizing the drug's side effects observed with systemic delivery. Strikingly the inventors could show that the extended release was achieved for up to three months with the microspheres of present invention after administration of only a single dose.

The present in invention therefore also relates to the biodegradable microsphere, the plurality of biodegradable microspheres, or the injectable formulation, as described herein, being administered intra-articularly, preferably wherein said microspheres, plurality of microspheres, or injectable formulation is being administered as a single dose.

The present invention furthermore relates to a method for treating a joint-related disorder in a subject comprising introducing the biodegradable microspheres, the plurality of biodegradable microspheres, or the injectable formulation as described herein into target tissue in or around one or more of the subject's joints, preferably the hips, shoulders and/or knees.

In one embodiment the subject is a human or an animal, such as a cat, a dog, or a horse.

In a further embodiment the joint-related disorder is arthritis, preferably osteoarthritis.

In one embodiment said method comprises intra-articularly injecting the biodegradable, the plurality of biodegradable microspheres, or the injectable formulation as described herein into one or both of the subject's knees, or shoulders, the hips, neck, lower back, or small joints of the hand.

It is to be understood that the joints as referred to in the description of present invention relate to any joints that can be affected by joint-related disorders, such as arthritis. Specifically, the joints can be joints of the knees, hips, shoulders, neck, lower back, small joints of the hand or feet.

KIT

In one aspect the present invention relates to a kit comprising, in separate compartments,

(a) a diluent, and

(b) biodegradable microspheres, or a plurality of biodegradable microspheres according to any one of the preceding claims.

The injectable formulation as disclosed herein can be provided as a single dose kit containing one vial of the microspheres comprising fenofibrate as disclosed herein. The microspheres can be provided in powder form increasing the storage stability of the active ingredient and the microspheres comprising it. The second vial provided in the kit can contain diluent which can be added to the first vial with the microspheres shortly before use/administration of the injectable formulation.

METHOD OF PREPARING THE MICROSPHERES

In one aspect the present invention relates to a method for preparing the microspheres as described herein, wherein the method comprises single emulsion-solvent evaporation. The single emulsion-solvent evaporation is a method that provides microspheres with the desired properties as described herein above as and that is at the same time extremely simple, economic and reliable with an easy scale-up. Furthermore, this method does not require extreme conditions avoiding the risk of degradation of the fenofibrate while ensuring an adequate drug encapsulation. Whilst single emulsion-solvent evaporation is well-known to the skilled person and can be carried out in the ways known in the prior art, examples 1 and 2 herein provide one way of applying said method in order to prepare the microspheres of present invention.

In a preferred embodiment of said method the microspheres are prepared with polymeric concentrations from about 10 - 30 % (w/w), preferably 15 - 25% (w/w), more preferably 15 - 20 % (w/w) of PLGA matrix.

In one embodiment of the method of the present invention, the method allows for the encapsulation of at least 90%, at least 91%, at least 92%, at least 93%, at least 94%, at least 95%, at least 96%, at least 97%, at least 98%, at least 99% of the fenofibrate into the microspheres of the present invention. The combination of the polymeric matrix used together with the use of the single emulsion-solvent evaporation method is preferred and allows for the high encapsulation rate as shown herein.

**EXAMPLES**

Example 1: Development of a long-term release formulation with microspheres and fenofibrate

*1. Materials*

PLGAs of variable lactic:glycolic ratio (L:G ratio) and molecular weight with the characteristics depicted is Table 1 were purchased from Evonik, Ltd. (Essen, Germany). Polyvinyl alcohol (PVA) (87-90% hydrolyzation, Av. Mol. Wt.

30,000-70,000 Da) was obtained from Sigma-Aldrich (St. Louis, USA). Dichloromethane (DCM) of high-performance liquid chromatography grade was purchased from Merck (Germany). All experiments were carried out using Milli-Q® water.

**Table 1.** Polymer characteristics according to the supplier specification sheets.

| Brand Name | Viscosity (dl/g) | End group | L:G ratio | Mw (Da) |
|---|---|---|---|---|
| Resomer® RG 502H | 0.21 | Acid | 50:50 | 13500 |
| Resomer® RG 503H | 0.38 | Acid | 50:50 | 30500 |
| Resomer® RG 752H | 0.18 | Acid | 75:25 | 11800 |
| Resomer® RG 753H | 0.39 | Acid | 75:25 | 31700 |

*2. Microspheres synthesis*

Fenofibrate loaded microspheres were obtained by a single emulsion-solvent evaporation method (o/w). For this purpose, a single PLGA polymer (Resomer® RG 502H or Resomer® RG 503H or Resomer® RG 752H or Resomer® RG 753H) was used. To obtain fenofibrate loaded microspheres 2mg of fenofibrate were dissolved in 200 μL of DCM. This solution was added to 0.8 mL of the respective PLGAs solution in DCM at 20% w/v and homogenized in a vortex obtaining the oil phase. Then, 4mL of an aqueous solution prepared with 1% PVA was added to the oil phase and homogenized at 1000 rpm for 5 minutes employing a magnetic stirrer to form the emulsion. Finally, the solvent was evaporated by pouring the obtained microspheres in 6 mL of PVA at 0.2% for twelve hours under continuous stirring to reach complete dichloromethane evaporation. Finally, the microspheres were washed trice with double distilled water, filtered through a 0.45 μm pore size filter (Pall Corporation, Sigma-Aldrich), freeze-dried and stored at 4°C until use.

*3. Microparticles characterization*

Microparticles were characterized in terms of size and size distribution by light diffraction employing a Mastersizer (Mastersizer 2000, Malvern Instruments, Malvern, UK). To determine the microspheres encapsulation efficiency (EE), 3 mg of microparticles were dissolved in 3 mL of dichloromethane, and centrifuged at 12,000 rpm for 30 minutes at 4°C. The drug concentration in the supernatant was determined using a validated spectrophotometric method. The EE was calculated using the following equations:

$$EE\ (\%) = \left[ \frac{actual\ drug\ content}{theoretical\ drug\ content} \right] x\ 100 \qquad (1)$$

*4. Fenofibrate drug release profiles*

Drug release studies were performed in simulated synovial fluid supplemented with 1% SDS to ensure sink conditions. Simulated synovial fluid was prepared following the procedure described by Marques et al and [1]. A known weight of microspheres (13 mg) was placed in 4 mL of the release media and incubated in a shaking bath at 37°C and 45 rpm for three months. At pre-set time points samples were centrifuged at 1,200rpm for 4 minutes and 2 ml of the supernatant was collected and replaced by fresh media. Samples were then again homogenized with a vortex and placed back in the shaking bath. The amount of fenofibrate released was quantified in the collected supernatant at 291 nm using a validated spectrophotometric method.

Example 2: Preparation of Fenofibrate loaded PLGA microspheres using PLGAs of different Mw

*1. Materials*

PLGAs of variable lactic:glycolic ratio (L:G ratio) and molecular weight with the characteristics depicted in Table 2 were purchased from Evonik, Ltd. (Essen, Germany). Polyvinyl alcohol (PVA) (87-90% hydrolyzation, Av. Mol. Wt. 30,000-70,000 Da) was obtained from Sigma-Aldrich (St. Louis, USA). Dichloromethane (DCM) of high-performance liquid chromatography grade was purchased from Merck (Germany). All experiments were carried out using Milli-Q® water.

**Table 2.** Polymer characteristics according to the supplier specification sheets.

| Brand Name | Viscosity (dl/g) | End group | L:G ratio | Mw (Da) |
|---|---|---|---|---|
| Resomer® RG 502H | 0.21 | Acid | 50:50 | 13500 |
| Resomer® RG 755S | 0.60 | Ester | 75:25 | 68000 |

*2. Microspheres synthesis*

Fenofibrate loaded microspheres were obtained by a single emulsion-solvent evaporation method (o/w) similarly as described in Example 1. For this purpose, PLGAs of variable molecular weight and L:G ratios were combined at different proportions to obtain the desired microspheres with improved properties: small size, narrow size distribution, high loading efficiency and adequate fenofibrate release profile. More specifically, Resomer® RG 502H was combined with Resomer® RG 755S at 75:25, 80:20 and 90:10 weight: weight ratios. To obtain fenofibrate loaded microspheres 0.2 or 2mg of fenofibrate were dissolved in 200 $\mu$L of DCM. This solution was added to 0.8 mL of the PLGAs solution in DCM at 20% w/v and homogenized in a vortex obtaining the oil phase. Then, 4mL of an aqueous solution prepared with 1% PVA was added to the oil phase and homogenized for one minute in a vortex at maximum speed. Finally, the solvent was evaporated by pouring the obtained microspheres in 96 mL of PVA at 0.16% for two hours under continuous stirring. After solvent removal, the microspheres were washed trice with double distilled water, filtered through a 0.45 $\mu$m pore size filter (Pall Corporation, Sigma-Aldrich), freeze-dried and stored at 4°C until use.

*3. Microparticles characterization*

Microparticles were characterized in terms of size and size distribution by light diffraction employing a Mastersizer (Mastersizer 2000, Malvern Instruments, Malvern, UK) as described in Example 1.

*4. Fenofibrate drug release profiles*

Drug release studies were performed in simulated synovial fluid as described for Example 1.

<u>Example 3: Efficacy of extended release of Fenofibrate in microspheres by intra-articular injection in a preclinical model of Osteoarthritis in mice</u>

The efficacy of the novel formulation of microspheres for controlled release of fenofibrate was evaluated on joint tissues by employing a surgically induced osteoarthritis model in mice. This is a predictive preclinical model of disease that resembles human pathological joint changes occurring in the cartilage and synovium.

*1. Surgically induced Osteoarthritis*

All animal experiments were performed according to protocols approved by the Institutional Animal Care and Use Committee at Instituto de Investigación Biomédica de A Coruña (INIBIC). Experimental OA was induced in 3-month-old male C57Bl/6J mice by transection of the medial meniscotibial ligament and the medial collateral ligament (MMTL+MCL) in the right knee as previously described [2]. The left knee was not be subjected to surgery, only open the joint capsule, and was used as a sham control. Body weight was evaluated every two weeks. Mice were euthanized 10 weeks after surgery.

*2. Extended release of Fenofibrate in microspheres by intra-articular administration*

FN was administered by a single intra-articular injection at two concentrations (1 and 10$\mu$g) of drug loaded microspheres. The extended-release formulation was prepared to allow release gradually into the joint for at least three months. To evaluate efficacy, three study groups were used. **Group 1:** mice treated with empty microspheres as placebo (Control group); **Group 2:** mice treated with FN-1$\mu$g; **Group 3:** mice treated with FN 10$\mu$g. Treatment was started one week after surgery recovery in all groups, meaning that the joint capsule had healed and closed before the intra-articular injection.

*3. Histological analysis of mouse knee joints*

Mice knee joint were fixed in 10% zinc-buffered formalin for 24 hours, decalcified in TBD for 6 hours, followed by paraffin embedding. Serial sections (4 $\mu$m) were cut, stained with Safranin O- fast green, and examined for histopathological changes using a semiquantitative scoring system for preclinical evaluation of whole joint changes according to OARSI guidelines [3]. In this system the scores are defined as follows: 0=normal cartilage, 0.5=loss of proteoglycan with an intact surface, 1=superficial fibrillation without loss of cartilage, 2=vertical clefts and loss of surface lamina (any % or joint surface area), 3=vertical clefts/ erosion to the calcified layer lesion for 1-25% of the quadrant width, 4=lesion reaches the calcified cartilage for 25-50% of the quadrant width, 5=lesion reaches the calcified cartilage for 50-75% of the quadrant width, 6=lesion reaches the calcified cartilage for >75% of the quadrant width.

*4. Statistical analysis*

Differences between two groups will be determined by Student's t-test, while differences between multiple groups will be determined by ANOVA with Tukey's multiple comparison. Data analysis will be performed by using Prism 9.0 software. Results will be reported as the mean $\pm$ SEM. p values <0.05 will be considered significant.

Example 4: Efficacy of extended release of Fenofibrate in microspheres by intra-articular injection in a preclinical model of Osteoarthritis in mice

The efficacy of the novel formulation of microspheres for controlled release of fenofibrate was evaluated on joint tissues by employing a surgically induced osteoarthritis model in mice. This is a predictive preclinical model of disease that resembles human pathological joint changes occurring in the cartilage and synovium.

*1. Surgically induced Osteoarthritis*

All animal experiments were performed according to protocols approved by the Institutional Animal Care and Use Committee at Instituto de Investigación Biomédica de A Coruña (INIBIC). Experimental OA was induced in 3-month-old male C57Bl/6J mice by transection of the lateral meniscotibial ligament and the lateral collateral ligament (LMTL+LCL) in the right knee as previously described [2]. The left knee was not be subjected to surgery, only open the joint capsule, and was used as a sham control. Body weight was evaluated every two weeks. Mice were euthanized 10 weeks after surgery.

*2. Extended release of Fenofibrate in microspheres by intra-articular administration*

FN was administered by a single intra-articular injection at two concentrations (1 and 10$\mu$g) of drug loaded microspheres. The extended-release formulation was prepared to allow release gradually into the joint for at least three months. To evaluate efficacy, three study groups were used. **Group 1:** mice treated with empty microspheres as placebo (Control group); **Group 2:** mice treated with FN-1$\mu$g; **Group 3:** mice treated with FN 10$\mu$g. Treatment was started one week after surgery recovery in all groups.

*3. Histological analysis of mouse knee joints*

Histopathological joint changes were evaluated as described in Example 3.

*4. Statistical analysis*

Statistical analysis was determined as described in Example 3.

Example 5: Efficacy of oral Fenofibrate in a preclinical model of surgically induced Osteoarthritis

*1. Surgically induced Osteoarthritis*

All animal experiments were performed according to protocols approved by the Institutional Animal Care and Use Committee at Instituto de Investigación Biomédica de A Coruña (INIBIC). Experimental OA in mouse knee joints was performed as described in Example 3.

*2. Treatment of Fenofibrate by oral administration*

FN was administered daily in the drinking water at 100 mg/kg body weight/day. To evaluate efficacy, two study groups were performed. **Group 1:** mice treated with vehicle (DMSO); **Group 2:** mice treated with FN 100 mg/kg body weight/day. Treatment was started three days after surgery recovery in all groups.

*3. Histological analysis of mouse knee joints*

Histopathological joint changes were evaluated as described in example 3.

*4. Statistical analysis*

Statistical analysis was determined as described in Example 3.

**List of References:**

[1] Marques, M. R. C., Loebenberg, R., & Almukainzi, M. (2011). Simulated Biological Fluids with Possible Application in Dissolution Testing. Dissolution Technologies, 18(3), 15-28. https://doi.org/10.14227/DT180311P15

[2] Caramés B, Hasegawa A, Taniguchi N, et al. Autophagy activation by rapamycin reduces severity of experimental osteoarthritis. Ann Rheum Dis. 2012; 71:575-81. doi: 10.1136/annrheumdis-2011-200557.

[3] Glasson SS, Chambers MG, Van Den Berg WB, et al. The OARSI histopathology initiative - recommendations for histological assessments of osteoarthritis in the mouse. Osteoarthritis Cartilage. 2010;18:S17-23. doi: 10.1016/j.joca.2010.05.025.

**Claims**

1.  A biodegradable microsphere, wherein the microsphere

    (i) comprises a polylactic-co-glycolic acid copolymer (PLGA) matrix, wherein said matrix comprises at least two PLGAs with different molecular weight (Mw);
    (ii) has a diameter of from about 25 $\mu$m to about 110 $\mu$m;
    (iii) comprises fenofibrate; and
    (iv) when present in the target tissue, releases fenofibrate for at least one month.

2.  The biodegradable microsphere of claim 1, wherein the at least two PLGAs each have a viscosity from about 0.16 dl/g to about 1.70 dl/g, preferably wherein one of the said at least two PLGAs has a viscosity from about 0.50 dl/g to about 0.70 dl/g and the second one of the at least two PLGAs has a viscosity from about 0.16 dl/g to about 0.24 dl/g.

3.  The biodegradable microsphere of claims 1 or 2, wherein the at least two PLGAs have a Mw between about 6 kDa and 90 kDa, preferably wherein one of the said at least two PLGAs has a Mw of between about 6 kDa and 18 kDa and the second one of the at least two PLGAs has a Mw of between about 50 kDa and 90 kDa.

4.  The biodegradable microsphere of any one claims 1 to 3, wherein one of the said at least two PLGAs have an average lactic acid to glycolic acid molar ratio from about 100:0 to about 50:50, preferably wherein one of the at least two PLGAs has an average lactic acid to glycolic acid molar ratio of about 75:25 (PLGA 75:25) and the second one of the at least two PLGAs has an average lactic acid to glycolic acid molar ratio of about 50:50 (PLGA 50:50).

5.  The biodegradable microsphere of claim 4, wherein the ratio between PLGA 50:50 and PLGA 75:25 is selected from about 90:10, about 80:20 or about 75:25, preferably from about 90:10.

6.  The biodegradable microsphere of any one of claims 1 to 5, wherein the diameter of the microsphere is between about 25 $\mu$m to about 110 $\mu$m, about 30 $\mu$m to about 100 $\mu$m, about 40 $\mu$m to about 90 $\mu$m, about 45 $\mu$m to about 80 $\mu$m, about 50 $\mu$m to about 70 $\mu$m, most preferred about 55 $\mu$m to about 65 $\mu$m.

7. The biodegradable microsphere of any one of claims 1 to 6, wherein the microsphere comprises from about 0.5 μg to about 20 μg, preferably from about 0.8 μg to about 15 μg, most preferred from about 1 μg to about 10 μg of fenofibrate per milligram of microsphere.

8. The biodegradable microsphere of any one of claims 1 to 7, wherein the microsphere

    (i) comprises a polylactic-co-glycolic acid copolymer (PLGA) matrix, wherein said matrix comprises PLGA 75:25 with a viscosity of about 0.60 dl/g and/or a Mw of about 68 kDa and PLGA 50:50 with a viscosity of about 0.21 dl/g and/or a Mw of about 13.5 kDa
    (ii) has a diameter of about 60 μm;
    (iii) comprises about 0.5 μg to about 15 μg fenofibrate per milligram of microsphere, preferably about 1 μg to about 10 μg fenofibrate per milligram of microsphere; and
    (iv) when present in the target tissue, releases fenofibrate for at least two months, more preferably for at least three months.

9. A plurality of biodegradable microspheres as defined in any one of the preceding claims, wherein the microspheres have a

    (i) d90 particle size value between about 50 μm and about 100 μm; and/or
    (ii) d10 particle size values between about 16 μm and about 20 μm; and/or
    (iii) d50 particle size values between about 30 μm and about 60 μm.

10. An injectable formulation comprising a pharmaceutically acceptable carrier and the microspheres or a plurality of biodegradable microspheres according to any one of the preceding claims.

11. The formulation of claim 10, wherein the microspheres, when present in the target tissue, release fenofibrate for at least one month, preferably for at least two months, more preferably for at least three months.

12. The biodegradable microsphere, the plurality of biodegradable microspheres, or the injectable formulation according to any one of the preceding claims for use in the treatment or prevention of a joint-related disorder, preferably arthritis, more preferably osteoarthritis.

13. The use of claim 12, wherein the biodegradable microsphere, the plurality of biodegradable microspheres, or the injectable formulation according to any one of the preceding claims is administered intra-articularly, preferably wherein said microspheres, plurality of microspheres, or injectable formulation are administered as a single-dose.

14. A kit comprising, in separate compartments,

    (a) a diluent, and
    (b) biodegradable microspheres, or a plurality of biodegradable microspheres according to any one of the preceding claims.

15. A method for preparing the microspheres according to any one of claims 1 to 8, wherein the method comprises single emulsion-solvent evaporation, preferably wherein the microspheres are prepared with polymeric concentrations from about 10 - 30 % (w/w), preferably 15 - 25% (w/w), more preferably 15 - 20 % (w/w) of PLGA matrix.

FIG.1

FIG.2

FIG.3

FIG.4

FIG.5

FIG.6

FIG.7

A)

B)

FIG.8

A)

B)

**Fig.9**

**A)**

**B)**

Europäisches
Patentamt

European
Patent Office

Office européen
des brevets

# EUROPEAN SEARCH REPORT

Application Number

EP 22 38 2101

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | KLOSE D ET AL:  "Fenofibrate-loaded PLGA microparticles: Effects on ischemic stroke", EUROPEAN JOURNAL OF PHARMACEUTICAL SCIENCES, ELSEVIER AMSTERDAM, NL, vol. 37, no. 1, 11 April 2009 (2009-04-11) , pages 43-52, XP025992155, ISSN: 0928-0987, DOI: 10.1016/J.EJPS.2008.12.016 [retrieved on 2009-01-09] * abstract * * page 44, right-hand column, paragraphs 2.1, 2.2 * * page 45, right-hand column, paragraph 2.9 * * page 48, left-hand column, last line, paragraph 3.1 * ----- | 1-15 | INV. A61K9/00 A61K9/16 A61K31/216 |
| A | US 2020/297651 A1 (MA JIAN-XING [US] ET AL) 24 September 2020 (2020-09-24) * example 2; table 3 * ----- | 1-15 | |
| | -/-- | | TECHNICAL FIELDS SEARCHED (IPC) A61K |

The present search report has been drawn up for all claims

| Place of search | Date of completion of the search | Examiner |
|---|---|---|
| Munich | 21 July 2022 | Madalinska, K |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 3

## EUROPEAN SEARCH REPORT

Application Number

EP 22 38 2101

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | QIU FANGFANG ET AL: "Fenofibrate-Loaded Biodegradable Nanoparticles for the Treatment of Experimental Diabetic Retinopathy and Neovascular Age-Related Macular Degeneration", MOLECULAR PHARMACEUTICS, [Online] vol. 16, no. 5, 26 March 2019 (2019-03-26) , pages 1958-1970, XP055932182, US ISSN: 1543-8384, DOI: 10.1021/acs.molpharmaceut.8b01319 Retrieved from the Internet: URL:https://pubs.acs.org/doi/pdf/10.1021/a cs.molpharmaceut.8b01319> * abstract * * page 1959, right-hand column, paragraph 2-4; table 2 * ----- | 1-15 | |
| A | CN 1 302 766 C (UNIV TONGJI [CN]) 7 March 2007 (2007-03-07) * example 9 * ----- | 1-15 | TECHNICAL FIELDS SEARCHED (IPC) |
| A | CN 100 400 032 C (UNIV TONGJI [CN]) 9 July 2008 (2008-07-09) * example 19 * ----- | 1-15 | |
| A | US 2015/164815 A1 (LOO SAY CHYE JOACHIM [SG] ET AL) 18 June 2015 (2015-06-18) * figure 15B; examples 1-2 * ----- -/-- | 1-15 | |

The present search report has been drawn up for all claims

| Place of search | Date of completion of the search | Examiner |
|---|---|---|
| Munich | 21 July 2022 | Madalinska, K |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

**Application Number**

**EP 22 38 2101**

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Grabacka M ET AL: "Fenofibrate subcellular distribution as a rationale for the intracranial delivery through biodegradable carrier", Journal of physiology and pharmacology : an official journal of the Polish Physiological Society, 1 April 2015 (2015-04-01), pages 233-247, XP055938185, Poland Retrieved from the Internet: URL:https://www.ncbi.nlm.nih.gov/pmc/artic les/PMC5865398/pdf/nihms951348.pdf [retrieved on 2022-07-04] * abstract; figures 3,9 * * page 5, paragraph 4 Preparation of poly(glycolide-co-lactide) wafers * * page 9, paragraph 3 * | 1-15 | |
| | ----- | | |
| A,D | NOGUEIRA-RECALDE UXÍA ET AL: "Fibrates as drugs with senolytic and autophagic activity for osteoarthritis therapy", EBIOMEDICINE, [Online] vol. 45, 5 July 2019 (2019-07-05), pages 588-605, XP055938558, NL ISSN: 2352-3964, DOI: 10.1016/j.ebiom.2019.06.049 Retrieved from the Internet: URL:https://www.sciencedirect.com/science/ article/pii/S235239641930430X/pdfft?md5=93 1c63056b790bc27dcd6217e7f3199d&pid=1-s2.0- S235239641930430X-main.pdf> [retrieved on 2022-07-06] * abstract; table 1 * * page 599, right-hand column, paragraph 3.7 - page 601, right-hand column, last paragraph; figure 10 * | 1-15 | **TECHNICAL FIELDS SEARCHED (IPC)** |
| | ----- | | |

The present search report has been drawn up for all claims

| Place of search | Date of completion of the search | Examiner |
|---|---|---|
| Munich | 21 July 2022 | Madalinska, K |

EPO FORM 1503 03.82 (P04C01)

page 3 of 3

## ANNEX TO THE EUROPEAN SEARCH REPORT
## ON EUROPEAN PATENT APPLICATION NO.

EP 22 38 2101

This annex lists the patent family members relating to the patent documents cited in the above-mentioned European search report.
The members are as contained in the European Patent Office EDP file on
The European Patent Office is in no way liable for these particulars which are merely given for the purpose of information.

21-07-2022

| Patent document cited in search report | | Publication date | Patent family member(s) | | Publication date |
|---|---|---|---|---|---|
| US 2020297651 | A1 | 24-09-2020 | NONE | | |
| CN 1302766 | C | 07-03-2007 | NONE | | |
| CN 100400032 | C | 09-07-2008 | NONE | | |
| US 2015164815 | A1 | 18-06-2015 | SG 11201500227S | A | 27-02-2015 |
| | | | US 2015164815 | A1 | 18-06-2015 |
| | | | WO 2014027956 | A1 | 20-02-2014 |

For more details about this annex : see Official Journal of the European Patent Office, No. 12/82

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MARQUES, M. R. C. ; LOEBENBERG, R. ; ALMUKAINZI, M.** *Simulated Biological Fluids with Possible Application in Dissolution Testing. Dissolution Technologies,* 2011, vol. 18 (3), 15-28, https://doi.org/10.14227/DT180311P15
- **CARAMÉS B ; HASEGAWA A ; TANIGUCHI N et al.** *Autophagy activation by rapamycin reduces severity of experimental osteoarthritis. Ann Rheum Dis.,* 2012, vol. 71, 575-81
- **GLASSON SS ; CHAMBERS MG ; VAN DEN BERG WB et al.** The OARSI histopathology initiative - recommendations for histological assessments of osteoarthritis in the mouse. *Osteoarthritis Cartilage,* vol. 2010 (18), 17-23